# EUROPEAN PATENT SPECIFICATION

(11) **EP 4 142 818 B1**
(45) Date of publication and mention of the grant of the patent: **17.06.2026**
(21) Application number: 21723056.4
(22) Date of filing: 08.04.2021
(51) Int. Cl.: A61L 31/16, A61L 31/10, A61F 2/00, B01J 23/06, B01J 23/50, B01J 23/72, B01J 23/745, B01J 27/057, C09D 101/08, B01J 37/02

(54) **NITRIC OXIDE INFUSED SURGICAL TISSUE**
MIT STICKSTOFFMONOXID INFUNDIERTES CHIRURGISCHES GEWEBE
TISSU CHIRURGICAL INFUSÉ D'OXYDE NITRIQUE

(30) Priority: 30.04.2020 US 202063018341 P; 07.04.2021 US 202117224942
(43) Date of publication of application: 08.03.2023
(73) Proprietor: Becton, Dickinson and Company, Franklin Lakes, NJ 07417 (US)
(72) Inventor: LAINE, Brendan, Salt Lake City, Utah 84113 (US); OFEK, Gidon, Millcreek, Utah 84109 (US)
(74) Representative: dompatent
(86) International application number: PCT/US2021/026467
(87) International publication number: WO 2021/221880

(56) References cited:
- US-A1- 2002 094 985
- US-A1- 2008 241 208
- SCOTT P. NICHOLS ET AL: "Local delivery of nitric oxide: Targeted delivery of therapeutics to bone and connective tissues", ADVANCED DRUG DELIVERY REVIEWS, vol. 64, no. 12, 10 March 2012 (2012-03-10), Amsterdam , NL, pages 1177 - 1188, XP055247724, ISSN: 0169-409X, DOI: 10.1016/j.addr.2012.03.002

## Description

### BACKGROUND

The present disclosure relates to surgical tissue repair technologies infused with nitric oxide. More specifically, the disclosure relates to incorporating nitric oxide releasing materials into biomedical technologies involved in surgical tissue repair, such as surgical meshes, vascular stents, surgical grafts, irrigation solutions, and other internal surgical tissue repair materials. Nitric oxide accelerates and enhances the tissue healing process by promoting reendothelialization, collagen deposition, and angiogenesis. Nitric oxide prevents infection by providing antimicrobial activity.

Surgical mesh includes sterile woven material designed for implantation within the body during open or laparoscopic surgical procedures. Surgical mesh is commonly used in hernia surgery and pelvic surgery. A wide range of mesh implants are available to stabilize and strengthen soft tissue defects and to act as a sling to support prolapsed organs and viscera. In tissue repair applications, the mesh serves to mechanically strengthen the weakened area while simultaneously promoting long-term stability by acting as a scaffold for new tissue growth.

Current issues associated with hernia repair meshes, for example, are inflammation, fibrosis, infection, mesh rejection, and hernia recurrence. It is difficult to find a mesh that mitigates all of these issues and that maintains sufficient tensile strength and elasticity. As of 2017 there were over 70 different hernia repair meshes commercially available, including synthetic non-absorbable, composite, and biological prosthesis. Synthetic meshes, such as polypropylene (PP) and polytetrafluoroethylene (PTFE), are often associated with chronic inflammatory responses, adhesion risks, and infection risks. Composite and absorbable meshes are associated with hernia recurrence, infection risk, risk of visceral adhesions, and low tensile strength. Lastly, biological meshes generally offer attractive biological responses with no inflammatory reaction, however, market acceptance is stifled by high costs.

Surgical grafts are devices similar to surgical meshes intended for implantation to reinforce soft tissue where weakness exists and for surgical repair of damaged or ruptured soft tissue. A non-limiting example of a surgical graft material the XenMatrix^{™} surgical graft material, provided by Becton, Dickinson and Company, which is an acellular non-crosslinked porcine collagen scaffold.

A stent is a metal or plastic device inserted into the lumen of an anatomic vessel or duct to keep the passageway open. Stent thrombosis is a rare but serious complication, which usually occurs in the first month after implantation. Associated factors include delayed endothelialization, and hypersensitivity reaction to stent coating drugs. Dual antiplatelet therapy is administered currently as a means to reduce thrombosis. This therapy is considered a predisposing factor for bleeding events occurring in patients following coronary stent implantation. There is a clear need in the art to reduce thrombotic potential and limit the need for dual antiplatelet therapy.

US 2002/094985 Al discloses medical articles useful for the local delivery of nitric oxide, and which comprise at least two differing nitric oxide donor compounds, comprising a first element comprising a first nitric oxide donor compound, and a second element comprising a second nitric oxide donor compound, the nitric oxide donor compounds being adsorbed to, attached to or disposed within compositions. US 2008/241208 Al discloses a drug delivery medical device able to promote angiogenesis by generating nitric oxide at a tissue locus, or enhance endothelial repair at site of vascular injury.

It would be an advancement in the art to provide surgical tissue repair technologies that accelerate and enhance the tissue healing process. It would be a further advancement in the art to provide surgical tissue repair technologies that promote reendothelialization, collagen deposition, and angiogenesis. It would be yet another advancement in the art to provide surgical tissue repair technologies that prevent infection by providing antimicrobial activity.

The background above is only provided to illustrate one example technology area where some implementations described herein may be practiced.

### SUMMARY

The present disclosure relates generally to biomedical technologies involved in internal tissue repair, which release nitric oxide to accelerate or enhance the healing process. Nitric oxide released into surrounding tissue promotes reendothelialization, collagen deposition, and angiogenesis. Nitric oxide also prevents infection by providing antimicrobial activity.

The disclosed biomedical technologies include surgical tissue repair devices configured to be implanted during tissue repair surgery which release nitric oxide into the surrounding tissue, wherein the devices comprise a substrate incorporating a nitric oxide releasing compound. Further disclosed but not claimed are biomedical technologies including wound irrigation solutions which release nitric oxide into the surrounding tissue.

The invention concerns a surgical tissue repair device configured to be implanted during tissue repair surgery and which releases nitric oxide into the surrounding tissue, wherein the device comprises a substrate incorporating a nitric oxide releasing compound, characterized in that the substrate further incorporates a catalyst to facilitate release of nitric oxide. The device substrate may be fabricated of any physiologically compatible material that may be impregnated or coated with the nitric oxide releasing compound and release nitric oxide.

One disclosed embodiment involves incorporating one or more nitric oxide releasing compounds into polymeric materials that make up the surgical tissue repair device.

Another disclosed embodiment involves incorporating one or more nitric oxide releasing compounds into a polymer-based coating applied to the surgical tissue repair device. A coating may be applied to the surgical tissue repair device and then impregnated with the nitric oxide releasing compound. A coating containing the nitric oxide releasing compound may be applied to the surgical tissue repair device.

The surgical tissue repair device may be configured in any form useful for tissue repair surgery. Non-limiting examples of surgical tissue repair devices include a surgical mesh, a surgical graft, and a vascular stent.

The nitric oxide releasing compound may be a S-nitrosothiol compound. The nitric oxide releasing compound may be selected from s-nitroso-n-acetyl penicillamine (SNAP), s-nitrosoglutathione (GSNO), and mixtures thereof.

The nitric oxide releasing compound may be selected to react in the presence of a physiological fluid to release nitric oxide into the surrounding tissue. The physiological fluid may be selected from interstitial fluid and blood.

In addition to the nitric oxide releasing compound, the substrate of the surgical tissue repair device according to the invention further incorporates a catalyst to facilitate release of nitric oxide. The catalyst may be selected from copper, iron, zinc, selenium, and silver.

The substrate of the surgical tissue repair device may be a polymeric material impregnated with the nitric oxide releasing compound. The substrate may be coated with a polymer-based coating incorporating the same or different nitric oxide releasing compound. The substrate may be impregnated and coated with the nitric oxide releasing compound.

The polymer-based coating may include diazeniumdiolate groups (NONOate groups).

The polymer-based coating may include a polyethyleneimine cellulose NONOate polymer.

Various embodiments are listed below. It will be understood that the embodiments listed below may be combined not only as listed below, but in other suitable combinations in accordance with the scope of the claims.

According to the invention, a surgical tissue repair device is configured to be implanted during tissue repair surgery and which releases nitric oxide into the surrounding tissue, wherein the device comprises a substrate incorporating a nitric oxide releasing compound and a catalyst to facilitate release of nitric oxide.

In one or more embodiments, the nitric oxide releasing compound is a S-nitrosothiol compound.

In any embodiment herein, the nitric oxide releasing compound is selected from s-nitroso-n-acetyl penicillamine (SNAP), s-nitrosoglutathione (GSNO), and mixtures thereof.

In any embodiment herein, the nitric oxide releasing compound may react in the presence of a physiological fluid to release nitric oxide into the surrounding tissue. The physiological fluid may be selected from interstitial fluid and blood.

The substrate further incorporates a catalyst to facilitate release of nitric oxide. The catalyst may be selected from copper, iron, zinc, selenium, and silver.

In any embodiment herein, the substrate may comprise a surgical mesh.

In any embodiment herein, the substrate may comprise a surgical graft.

In any embodiment herein, the substrate may comprise a vascular stent.

In any embodiment herein, the substrate may be a polymeric material impregnated with the nitric oxide releasing compound.

In any embodiment herein, the substrate may be coated with a polymer-based coating incorporating the same or different nitric oxide releasing compound. The polymer-based coating may comprise diazeniumdiolate groups (NONOate groups). The polymer-based coating may be a polyethyleneimine cellulose NONOate polymer.

In any embodiment herein, the substrate may be a polymeric material impregnated with the nitric oxide releasing compound and coated with a polymer-based coating incorporating the same or different nitric oxide releasing compound.

It is to be understood that both the foregoing general description and the following detailed description are examples and explanatory and are not restrictive of the invention, as claimed.

### BRIEF DESCRIPTION OF THE SEVERAL VIEWS OF THE DRAWINGS

Example embodiments will be described and explained with additional specificity and detail through the use of the accompanying drawings in which:
Fig. 1 is cross-sectional representation of portion of a surgical tissue repair device fabricated to contain a nitric oxide releasing compound according to an embodiment.
Fig. 2 is cross-sectional representation of portion of a surgical tissue repair device fabricated to contain a nitric oxide releasing compound according to an embodiment.
Fig. 3 is cross-sectional representation of portion of a surgical tissue repair device fabricated to contain a nitric oxide releasing compound according to an embodiment.

### DESCRIPTION OF EMBODIMENTS

The disclosure relates to a biomedical technologies involved in internal surgical tissue repair which release nitric oxide to accelerate or enhance the healing process.

According to the invention, a surgical tissue repair device is configured to be implanted during tissue repair surgery and which releases nitric oxide into the surrounding tissue, wherein the device comprises a substrate incorporating a nitric oxide releasing compound and a catalyst to facilitate release of nitric oxide.

Any physiologically compatible nitric oxide releasing compound may be used herein. Non-limiting examples of nitric oxide releasing compounds include s-nitrosoglutathione (GSNO), s-nitroso-n-acetylpenicillamine (SNAP), and mixtures thereof. S-nitrosoglutathione (GSNO) and s-nitroso-n-acetylpenicillamine (SNAP) are naturally occurring and/or biocompatible materials that release in-vivo and degrade to form nitric oxide at physiologically relevant concentrations.

Nitric oxide is a messenger molecule expressed naturally in the inflammatory and proliferative phases of the wound healing process. It is responsible for promoting collagen synthesis and deposition, chemotaxis and reepithelialization, and angiogenesis. Additionally, it has anti-platelet activation properties-leading to a decrease in contact activated thrombogenesis.

Incorporating nitric oxide into wound repair technologies such as surgical meshes, surgical grafts, and vascular stents may reduce adverse inflammatory reactions and enhance adoption of foreign material into the surrounding tissues through increased angiogenesis and reepithelialization. Because of its anti-platelet activation characteristics, nitric oxide has the potential to reduce or eliminate the need for dual antiplatelet therapy following vascular stent implantation, which is associated with bleeding events following coronary stent implantation.

Lastly, nitric oxide has natural antimicrobial properties. At low concentrations it acts as a signaling molecule that promotes growth and activity of immune cells. At higher concentrations (respiratory burst of a neutrophil) NO covalently binds DNA, proteins and lipids of pathogens and kills them. There are also reactive species created by the auto-oxidation of nitric oxide, which directly react with DNA structure, inhibit DNA repair, and increase generation of alkylating agents and H₂O₂, which are genotoxic. These natural antimicrobial properties are an important aspect of the present disclosure since infections are a serious complication associated with all wound repair technologies.

The nitric oxide releasing compound may be impregnated in the surgical tissue repair device substrate, and particularly to surfaces of the device substrate that may be exposed to blood and interstitial fluid.

The impregnating step may be accomplished by exposing the surgical tissue repair device substrate to a solvent having the nitric oxide releasing compound dissolved therein. The substrate is exposed to the solvent solution for sufficient time to permit the nitric oxide releasing compound to penetrate the substrate. The impregnating step may occur at room temperature. The impregnating step may occur at a temperature in the range from about 20 to 60 °C. Any solvent that is compatible with the nitric oxide releasing compound and device substrate may be used. The nitric oxide releasing compound may be dissolved in tetrahydrofuran (THF), dioxolane, methyl ethyl ketone (MEK), methanol, ethanol, isopropyl alcohol, water, or combinations thereof. The device substrate may be soaked in these solutions containing the nitric oxide releasing compound for sufficient time to impregnate the device substrate with the nitric oxide releasing compound. The exposure time may range between 5 minutes and 24 hours.

The device substrate **further incorporates a catalyst to facilitate release of nitric oxide**. Non-limiting examples of such catalysts include copper, iron, zinc, selenium, and silver. The catalyst may be impregnated into the device substrate by exposing the device substrate to a solvent having the catalyst dissolved therein. The catalyst may be impregnated into the device substrate using the same solvent system as the nitric oxide releasing compound, discussed above, either during the same impregnation step, a subsequent impregnation step, or a prior impregnation step. The device substrate is exposed to the solvent solution for sufficient time to permit the catalyst to penetrate the device substrate. The impregnating step may occur at room temperature. The impregnating step may occur at a temperature in the range from about 20 to 60 °C. Any solvent that is compatible with the catalyst and device substrate may be used, including those described above in relation to the nitric oxide releasing compound.

Reference is made to the figures which illustrate portions of a surgical tissue repair device. The surgical tissue repair device may be configured in any form useful for tissue repair surgery. Non-limiting examples of surgical tissue repair devices include a surgical mesh, a surgical graft, and a vascular stent.

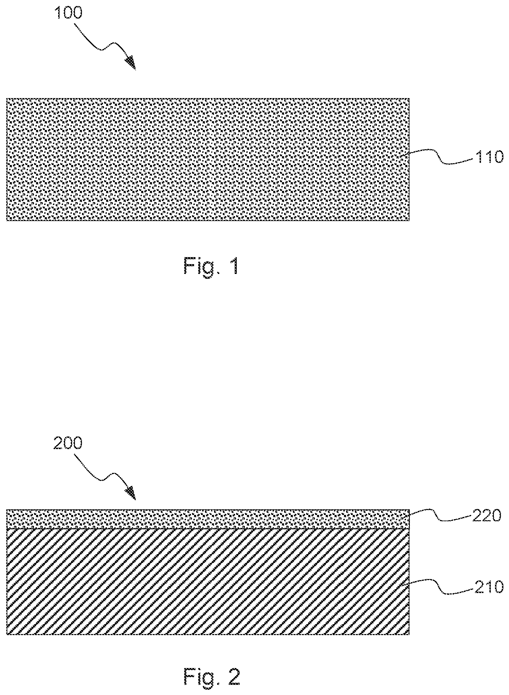

Fig. 1 is cross-sectional representation of a portion of a surgical tissue repair device 100 fabricated to contain a nitric oxide (NO) releasing compound. The tissue repair device 100 includes a device substrate 110 impregnated with a nitric oxide releasing compound.

The nitric oxide releasing compound may be impregnated in the device substrate 110 by exposing the device substrate 110 to a solvent having the nitric oxide releasing compound dissolved therein. The device substrate 110 is exposed to the solvent solution for sufficient time to permit the nitric oxide releasing compound to penetrate the device substrate 110. The impregnating step may occur at any suitable temperature. The impregnating step may occur at room temperature. The impregnating step may occur at a temperature in the range from about 20 to 60 °C. Any solvent that is compatible with the device substrate and the nitric oxide releasing compound may be used.

The nitric oxide releasing compound may be dissolved in tetrahydrofuran (THF), dioxolane, methyl ethyl ketone (MEK), methanol, ethanol, isopropyl alcohol, water, or combinations thereof. The device substrate may be soaked in these solutions containing the nitric oxide releasing compound for sufficient time to impregnate the device substrate with the nitric oxide releasing compound. The exposure time may range between 5 minutes and 24 hours.

The device substrate 110 may be further impregnated with a catalyst to facilitate release of nitric oxide. Non-limiting examples of such catalysts include copper, iron, zinc, selenium, and silver. The catalyst may be impregnated into the device substrate 110 by exposing the device substrate to a solvent having the catalyst dissolved therein. The catalyst may be impregnated into the device substrate using the same solvent system as the nitric oxide releasing compound, discussed above, either during the same impregnation step, a subsequent impregnation step, or a prior impregnation step. The device substrate is exposed to the solvent solution for sufficient time to permit the catalyst to penetrate the device substrate. The impregnating step may occur at any suitable temperature. The impregnating step may occur at room temperature. The impregnating step may occur at a temperature in the range from about 20 to 60 °C. Any solvent that is compatible with the device substrate and the catalyst may be used.

Fig. 2 is a cross-sectional representation of a portion of a surgical tissue repair device 200 fabricated to contain a nitric oxide (NO) releasing compound. The tissue repair device 200 includes a substrate 210 coated with a polymer-based coating 220 incorporating a nitric oxide releasing compound.

In one non-limiting embodiment, the coating 220 may be coated onto the substrate 210 and then infused or impregnated with one or more nitric oxide releasing compounds. The coating 220 may be infused or impregnated with a nitric oxide releasing compound by exposing the coating 220 to a solvent having the nitric oxide releasing compound dissolved therein, in a manner similar to the discussion of Fig. 1, above.

The coating 220 is exposed to the solvent solution for sufficient time to permit the nitric oxide releasing compound to penetrate the coating 220. The impregnating step may occur at any suitable temperature. The impregnating step may occur at room temperature. The impregnating step may occur at a temperature in the range from about 20 to 60 °C. Any solvent that is compatible with the coating and the nitric oxide releasing compound may be used.

The nitric oxide releasing compound may be dissolved in tetrahydrofuran (THF), dioxolane, methyl ethyl ketone (MEK), methanol, ethanol, isopropyl alcohol, water, or combinations thereof. The coating 220 may be soaked in these solutions containing the nitric oxide releasing compound for sufficient time to impregnate the coating with the nitric oxide releasing compound. The exposure time may range between 5 minutes and 24 hours.

The coating 220 may be further impregnated with a catalyst to facilitate release of nitric oxide. Non-limiting examples of such catalysts include copper, iron, zinc, selenium, and silver. The catalyst may be impregnated into the coating 220 by exposing the coating to a solvent having the catalyst dissolved therein. The catalyst may be impregnated into the coating using the same solvent system as the nitric oxide releasing compound, discussed above, either during the same impregnation step, a subsequent impregnation step, or a prior impregnation step. The coating is exposed to the solvent solution for sufficient time to permit the catalyst to penetrate the coating. The impregnating step may occur at any suitable temperature. The impregnating step may occur at room temperature. The impregnating step may occur at a temperature in the range from about 20 to 60 °C. Any solvent that is compatible with the coating and the catalyst may be used.

In one non-limiting embodiment, a polymer coating material incorporating one or more nitric oxide releasing compounds may be coated onto the substrate 210 to form coating 220.

Non-limiting examples of a polymer material incorporating one or more nitric oxide releasing compounds, which can release nitric oxide directly, include, but are not limited to, polymers containing diazeniumdiolate groups (NONOate groups) and polyethyleneimine (PEI) cellulose NONOate. NONOates carry an [N(O-)N=O] group on a nucleophile adduct, usually an amine. NONOates decompose spontaneously in solution at physiological pH and temperature to generate NO. The NONOate groups may be noncovalently dispersed within the polymer matrix, covalently bound to pendent polymer side chains, or covalently bound directly to the polymer backbone.

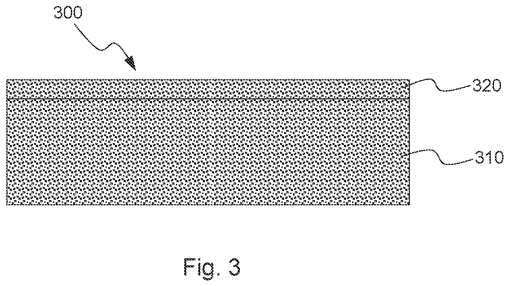

Fig. 3 is a cross-sectional representation of a portion of a surgical tissue repair device 300 fabricated to contain a nitric oxide (NO) releasing compound. The tissue repair device 300 includes a device substrate 310 impregnated with a nitric oxide releasing compound, similar to device substrate 110 described above. The device substrate 310 is further coated with a polymer-based coating 320 incorporating the same or a different nitric oxide releasing compound, similar to the coating 220 described above.

The words "including," "having," and variants thereof (e.g., "include," "includes," "have," and "has") as used in the present disclosure, including the claims, shall be open ended and have the same meaning as the word "comprising" and variants thereof (e.g., "comprise" and "comprises").

## Claims

1. A surgical tissue repair device (100, 200, 300) configured to be implanted during tissue repair surgery and which releases nitric oxide into the surrounding tissue, wherein the device comprises a substrate (110, 210, 310) incorporating a nitric oxide releasing compound,
**characterized in that**
the substrate further incorporates a catalyst to facilitate release of nitric oxide.

2. The surgical tissue repair device of claim 1, wherein the nitric oxide releasing compound is a S-nitrosothiol compound.

3. The surgical tissue repair device of claim 1, wherein the nitric oxide releasing compound is selected from s-nitroso-n-acetyl penicillamine (SNAP), s-nitrosoglutathione (GSNO), and mixtures thereof.

4. The surgical tissue repair device of claim 1, wherein the nitric oxide releasing compound reacts in the presence of a physiological fluid to release nitric oxide into the surrounding tissue.

5. The surgical tissue repair device of claim 4, wherein the physiological fluid is selected from interstitial fluid and blood.

6. The surgical tissue repair device of claim 1, wherein the substrate is impregnated with the catalyst.

7. The surgical tissue repair device of claim 1, wherein the catalyst is selected from copper, iron, zinc, selenium, and silver.

8. The surgical tissue repair device of claim 1, wherein the substrate comprises a surgical mesh.

9. The surgical tissue repair device of claim 1, wherein the substrate comprises a surgical graft.

10. The surgical tissue repair device of claim 1, wherein the substrate comprises a vascular stent.

11. The surgical tissue repair device of claim 1, wherein the substrate is a polymeric material impregnated with the nitric oxide releasing compound.

12. The surgical tissue repair device of claim 1, wherein the substrate is impregnated with the nitric oxide releasing compound.

13. The surgical tissue repair device of claim 1, wherein the substrate is coated with a polymer-based coating (220, 320) incorporating a different nitric oxide releasing compound.

14. The surgical tissue repair device of claim 13, wherein the polymer-based coating (220, 320) comprises diazeniumdiolate groups (NONOate groups) and/or a polyethyleneimine cellulose NONOate polymer.

15. The surgical tissue repair device of claim 13, wherein the polymer-based coating (220, 320) is impregnated with a catalyst to facilitate release of nitric oxide.

## Patentansprüche

1. Chirurgische Gewebereparaturvorrichtung (100, 200, 300), die so konfiguriert ist, dass sie während einer Gewebereparaturoperation implantiert werden kann und dann Stickstoffmonoxid in das umgebende Gewebe freisetzt, wobei die Vorrichtung ein Substrat (110, 210, 310) umfasst, in das eine Stickstoffmonoxid-freisetzende Verbindung eingebaut ist,
**dadurch gekennzeichnet, dass**
in das Substrat weiterhin ein Katalysator eingebaut ist, um die Freisetzung von Stickstoffmonoxid zu erleichtern.

2. Chirurgische Gewebereparaturvorrichtung gemäß Anspruch 1, wobei die Stickstoffmonoxid-freisetzende Verbindung eine S-Nitrosothiol-Verbindung ist.

3. Chirurgische Gewebereparaturvorrichtung gemäß Anspruch 1, wobei die Stickstoffmonoxid-freisetzende Verbindung aus S-Nitroso-N-acetylpenicillamin (SNAP), S-Nitrosoglutathion (GSNO) und Gemischen davon ausgewählt ist.

4. Chirurgische Gewebereparaturvorrichtung gemäß Anspruch 1, wobei die Stickstoffmonoxid-freisetzende Verbindung in Gegenwart einer physiologischen Flüssigkeit so reagiert, dass sie Stickstoffmonoxid in das umgebende Gewebe freisetzt.

5. Chirurgische Gewebereparaturvorrichtung gemäß Anspruch 4, wobei die physiologische Flüssigkeit aus Gewebsflüssigkeit und Blut ausgewählt ist.

6. Chirurgische Gewebereparaturvorrichtung gemäß Anspruch 1, wobei das Substrat mit dem Katalysator imprägniert ist.

7. Chirurgische Gewebereparaturvorrichtung gemäß Anspruch 1, wobei der Katalysator aus Kupfer, Eisen, Zink, Selen und Silber ausgewählt ist.

8. Chirurgische Gewebereparaturvorrichtung gemäß Anspruch 1, wobei das Substrat ein chirurgisches Netz umfasst.

9. Chirurgische Gewebereparaturvorrichtung gemäß Anspruch 1, wobei das Substrat ein chirurgisches Transplantat umfasst.

10. Chirurgische Gewebereparaturvorrichtung gemäß Anspruch 1, wobei das Substrat einen Stent umfasst.

11. Chirurgische Gewebereparaturvorrichtung gemäß Anspruch 1, wobei das Substrat ein polymeres Material ist, das mit der Stickstoffmonoxid-freisetzenden Verbindung imprägniert ist.

12. Chirurgische Gewebereparaturvorrichtung gemäß Anspruch 1, wobei das Substrat mit der Stickstoffmonoxid-freisetzenden Verbindung imprägniert ist.

13. Chirurgische Gewebereparaturvorrichtung gemäß Anspruch 1, wobei das Substrat mit einer Beschichtung auf Polymerbasis (220, 320) beschichtet ist, in die eine andere Stickstoffmonoxid-freisetzende Verbindung eingebaut ist.

14. Chirurgische Gewebereparaturvorrichtung gemäß Anspruch 13, wobei die Beschichtung auf Polymerbasis (220, 320) Diazeniumdiolat-Gruppen (NONOat-Gruppen) und/oder ein Polyethylenimin-Cellulose-NONOat-Polymer umfasst.

15. Chirurgische Gewebereparaturvorrichtung gemäß Anspruch 13, wobei die Beschichtung auf Polymerbasis (220, 320) mit einem Katalysator imprägniert ist, um die Freisetzung von Stickstoffmonoxid zu erleichtern.

## Revendications

1. Dispositif chirurgical de réparation tissulaire (100, 200, 300) configuré pour être implanté pendant une chirurgie de réparation de tissu et qui libère de l'oxyde nitrique dans le tissu environnant, dans lequel le dispositif comprend un substrat (110, 210, 310) incorporant un composé de libération d'oxyde nitrique,
**caractérisé en ce que**
le substrat incorpore en outre un catalyseur pour faciliter la libération d'oxyde nitrique.

2. Dispositif chirurgical de réparation tissulaire selon la revendication 1, dans lequel le composé de libération d'oxyde nitrique est un composé S-nitrosothiol.

3. Dispositif chirurgical de réparation tissulaire selon la revendication 1, dans lequel le composé de libération d'oxyde nitrique est choisi parmi la s-nitroso-n-acétyl pénicillamine (SNAP), la s-nitrosoglutathione (GSNO) et leurs mélanges.

4. Dispositif chirurgical de réparation tissulaire selon la revendication 1, dans lequel le composé de libération d'oxyde nitrique réagit en présence d'un fluide physiologique pour libérer de l'oxyde nitrique dans le tissu environnant.

5. Dispositif chirurgical de réparation tissulaire selon la revendication 4, dans lequel le fluide physiologique est choisi parmi le fluide interstitiel et le sang.

6. Dispositif chirurgical de réparation tissulaire selon la revendication 1, dans lequel le substrat est imprégné du catalyseur.

7. Dispositif chirurgical de réparation tissulaire selon la revendication 1, dans lequel le catalyseur est choisi parmi le cuivre, le fer, le zinc, le sélénium et l'argent.

8. Dispositif chirurgical de réparation tissulaire selon la revendication 1, dans lequel le substrat comprend un treillis chirurgical.

9. Dispositif chirurgical de réparation tissulaire selon la revendication 1, dans lequel le substrat comprend une greffe chirurgicale.

10. Dispositif chirurgical de réparation tissulaire selon la revendication 1, dans lequel le substrat comprend un stent vasculaire.

11. Dispositif chirurgical de réparation tissulaire selon la revendication 1, dans lequel le substrat est un matériau polymère imprégné du composé de libération d'oxyde nitrique.

12. Dispositif chirurgical de réparation tissulaire selon la revendication 1, dans lequel le substrat est imprégné du composé de libération d'oxyde nitrique.

13. Dispositif chirurgical de réparation tissulaire selon la revendication 1, dans lequel le substrat est revêtu d'un revêtement à base de polymère (220, 320) incorporant un composé de libération d'oxyde nitrique différent.

14. Dispositif chirurgical de réparation tissulaire selon la revendication 13, dans lequel le revêtement à base de polymère (220, 320) comprend des groupes diazéniumdiolate (groupes NONOate) et/ou un polymère de polyéthylèneimine-cellulose NONOate.

15. Dispositif chirurgical de réparation tissulaire selon la revendication 13, dans lequel le revêtement à base de polymère (220, 320) est imprégné d'un catalyseur pour faciliter la libération d'oxyde nitrique.
